# EUROPEAN PATENT APPLICATION

(11) **EP 4 269 575 A1**
(43) Date of publication of application: **01.11.2023**
(21) Application number: 21915387.1
(22) Date of filing: 26.04.2021
(51) Int. Cl.: C12N 9/10, C12N 15/70, C12P 13/24

(54) **ATP-PRT VARIANT WITH REDUCED FEEDBACK INHIBITION BY HISTIDINE, AND HISTIDINE-PRODUCING STRAIN EXPRESSING SAME**

(30) Priority: 28.12.2020 KR 20200184686
(71) Applicant: Daesang Corporation, Seoul 02586 (KR)
(72) Inventor: HAN, Jong Yun, Yongin-si, Gyeonggi-do 17048 (KR); YANG, Chel Min, Goyang-si, Gyeonggi-do 10474 (KR); KIM, Yong Soo, Yongin-si, Gyeonggi-do 17117 (KR); JO, Young Il, Seoul 04995 (KR)
(74) Representative: Cabinet Nony
(86) International application number: PCT/KR2021/005246
(87) International publication number: WO 2022/145588

(57) **Abstract**

The present invention relates to an *E. coli* hisG-derived ATP-phosphoribosyltransferase variant having a reduced feedback inhibition by histidine and a strain expressing the same. The variant may maintain its activity even at a high histidine concentration, thus increasing histidine production.

## Description

### Technical Field

The present invention relates to an ATP-PRT variant having a reduced feedback inhibition by histidine and a histidine-producing strain expressing the same.

### Background Art

ATP-phosphoribosyltransferase (which hereinafter may be referred to as ATP-PRT) catalyzes the first step of histidine biosynthesis in bacteria, fungi, or plants.

In an environment where a certain concentration or higher of L-histidine is present, a certain level because the activity of ATP-phosphoribosyltransferase is feedback-inhibited by histidine, and thus it is difficult to increase histidine production beyond.

Therefore, ATP-PRT variants having increased resistance to histidine feedback inhibition are required to increase histidine production in microorganisms. However, there is no known variant that can reduce histidine feedback inhibition of ATP-PRT that is expressed from the *E*. *coli* hisG gene.

### [Prior Art Documents]

### [Patent Documents]

(Patent Document 1) Korean Patent Application Publication No. 10-2017-0098205 (August 2, 2017)

### DISCLOSURE

### Technical Problem

According to one embodiment, there is provided an ATP-phosphoribosyltransferase variant having a reduced feedback inhibition by histidine.

### Technical Solution

One aspect provides an ATP-phosphoribosyltransferase variant containing a substitution of a substitution of proline for serine at position 288 in an ATP-phosphoribosyltransferase consisting of the amino acid sequence of SEQ ID NO: 1.

The amino acid sequence of SEQ ID NO: 1 is the sequence of ATP-phosphoribosyltransferase expressed from wild-type hisG of *Escherichia coli.* ATP-phosphoribosyltransferase may be referred to as ATP-PRT. ATP-PRT catalyzes the reaction 1-(5-phospho-D-ribosyl)-ATP + diphosphate ↔ ATP + 5-phospho-alpha-D-ribose 1-diphosphate which is the first step of histidine biosynthesis. In the present application, the term "ATP-phosphoribosyltransferase" may be used interchangeably with the term "hisG".

According to one embodiment, the ATP-phosphoribosyltransferase may be expressed from the *E. coli* hisG gene.

According to one embodiment, the variant may be reduced feedback inhibition of the variant by histidine. In one example, histidine production in a strain into which an ATP-PRT containing an S288P mutation has been introduced was higher than that in the wild type strain.

According to one embodiment, the variant may further contain at least one of: (a) a substitution of histidine for arginine at position 250; a substitution of lysine (K) or threonine (T) for histidine (H) at position 232; (b) (c) a substitution of alanine, leucine, glycine, valine or isoleucine for threonine at position 252; and (d) a substitution of lysine for glutamic acid at position 271. In one example, it was confirmed that histidine production increased when the variant further contained mutations (a) to (d) in addition to the serine mutation at position 288.

The variant may have activity even at a histidine concentration of 5 mM to 25 mM.

Another aspect provides a polynucleotide encoding the ATP-phosphoribosyltransferase variant, or a vector containing the same. The vector may be a plasmid or phage.

Still another aspect provides a transformed strain expressing the ATP-phosphoribosyltransferase variant. The transformed strain may be a strain into which a polynucleotide encoding the ATP-phosphoribosyltransferase variant, or a vector containing the same has been introduced. Since the strain maintains the activity of ATP-phosphoribosyltransferase even when the concentration of histidine increases, histidine production in the strain may increase.

Histidine production in the strain expressing the ATP-phosphoribosyltransferase variant may increase by about 22 to 92%.

The transformation may be performed by a known method, for example, an electroporation method (van der Rest et al., Appl. Microbiol. Biotechnol., 52, 541-545, 1999).

According to one embodiment, the strain may be a strain of the genus *Escherichia.* Specifically, the strain may be an *Escherichia coli, Escherichia albertii, Escherichia blattae, Escherichia fergusonii (Escherichia hermannii)* or *Escherichia vulneris* strain.

Still yet another aspect provides a method for producing histidine comprising a step of culturing the transformed strain. The method for producing histidine may comprise steps of: culturing the transformed strain in a medium; and recovering histidine from the strain or the medium.

The medium may contain carbon sources, nitrogen sources, and inorganic salts. Examples of the carbon sources include, but are not particularly limited to, saccharides and carbohydrates such as glucose, sugar, citrate, fructose, lactose, maltose or molasses; oils and fats, such as soybean oil, sunflower oil, castor oil or coconut oil; fatty acids such as palmitic acid, stearic acid or linoleic acid; glycerol; alcohols such as ethanol; and organic acids such as acetic acid, and these substances may be used individually or as a mixture. Preferably, the medium for the *Escherichia coli* mutant strain may contain glucose. Examples of the nitrogen sources that may be used in the medium include, but are not particularly limited to, peptone, meat extract, yeast extract, dried yeast, corn steep liquor, soybean cake, urea, thiourea, ammonium salt, nitrate, and other compounds containing organic or inorganic nitrogen. In addition, examples of inorganic salts that may be used in the medium include, but are not limited to, magnesium, manganese, potassium, calcium, iron, zinc, cobalt, etc.

Furthermore, to adjust the pH of the medium, a basic compound such as sodium hydroxide, potassium hydroxide or ammonia, or an acidic compound such as phosphoric acid or sulfuric acid may be used in an appropriate manner. In addition, foaming may be suppressed using an anti-foaming agent such as a fatty acid polyglycol ester. To keep an aerobic condition, oxygen or an oxygen-containing gas (for example, air) may be injected into the medium.

The culturing means growing a microorganism in an artificially controlled suitable environmental, and may be performed by a culturing method widely known in the art. The culturing may be performed at a temperature of 20 to 45°C for 10 to 200 hours, without being limited thereto.

The step of recovering histidine may be performed using various methods widely known in the art. For example, the step may be performed using centrifugation, filtration, anion exchange chromatography, crystallization, or HPLC, without being limited thereto.

### Advantageous Effects

The ATP-phosphoribosyltransferase variant according to one embodiment may maintain its activity even in a high-concentration histidine environment.

A strain expressing the ATP-phosphoribosyltransferase variant according to one embodiment is capable of producing an increased amount of histidine.

### Brief Description of Drawings

FIG. 1 shows the results of examining changes in the enzyme activities of *E*. *coli-*derived hisG_WT and variants thereof (hisG_SDM4 and hisG_SDM7) depending on the histidine concentration.
FIG. 2 shows computer simulation results for a mode of binding between a hisG hexamer and histidine. This indicates that H232, S288, T252, R250, A248, E271, and E240 of hisG interact with histidine.

### Mode for Invention

Hereinafter, one or more embodiments will be described in more detail with reference to examples. However, these examples are intended to illustrate one or more embodiments, and the scope of the present invention is not limited to these examples.

### Example 1: Selection of mutant strains having resistance to 1,2,4-triazole-3-alanine (TRA)

In order to construct mutant strains in which negative feedback by L-histidine has been reduced, the present inventors constructed mutant strains having resistance to 1,2,4-triazole-3-alanine (TRA), which is a derivative of L-histidine, using N-methyl-N'-nitro-N-nitrosoguanidine (NTG) which is a chemical mutagenesis agent.

*E. coli* MG1655 (KCTC14419BP) was cultured in LB medium for 16 hours (37°C and 200 rpm). After culturing, the cells were centrifuged at 4,500 rpm for 10 minutes and suspended in saline/TM buffer. After resuspending the cells in buffer, 100 ug/ml of NTG was added to the cells, and mutagenesis was performed at 37°C and 200 rpm for 30 minutes.

After repeating the mutagenesis process, the cells were suspended in 3 ml of deionized water, plated on a plate medium (composition: 8% glucose, 0.6% sodium monohydrogenphosphate, 0.2% ammonium sulfate, 0.02% magnesium sulfate, 0.001% calcium nitrate, 10 ppm iron sulfate, and 1% TRA), and subjected to primary culture at 37°C for 2 days. Strains forming single colonies were isolated and subjected to secondary culture on a plate medium supplemented with 1% TRA in the same manner as the primary culture, and mutant strains were selected.

The degrees of growth (increases in the number of cells) of the selected mutants on plate media supplemented with 0%, 0.5%, 1.0% or 2.0% TRA were measured and their resistances to TRA were compared (see Table 1 below).

**[Table 1]**

| | Degree of growth | | |
|---|---|---|---|
| TRA concentration | MG1655 | H-1 | H-2 |
| 0% | ++++ | ++++ | ++++ |
| 0.5% | ++ | +++ | +++ |
| 1.0% | - | + | ++ |
| 2.0% | - | - | + |

### Example 2: Analysis of amino acid sequences of ATP-PRT enzymes in TRA-resistant mutant strains

Amino acid sequences of ATP-phosphoribosyltransferase (hisG) enzymes in the mutant strains H-1 and H-2 having increased resistance to TRA were comparatively analyzed. Analysis of the sequences (Sequencing) was performed by Macrogen, and the sequences were analyzed using the primers shown in Table 2 below.

**[Table 2]**

| SEQ ID NO. | Primer | Nucleotide sequence (5'→3') |
|---|---|---|
| 9 | hisGW_CF | AGTTCATTGTACAATGATGAGCG |
| 10 | hisGW_CR | AGCCGCCAGGAATATACAAC |

As a result, it was confirmed that some of the amino acids at the C-terminal region of the ATP-PRT enzyme were substituted.

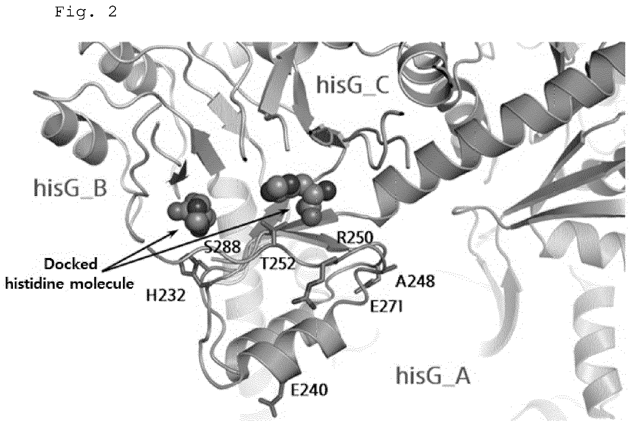

In addition, using an intermolecular binding mode prediction program, the three-dimensional structure of the *E. coli*-derived hisG hexamer upon docking with a histidine molecule was analyzed, and based on the results of the docking analysis, the amino acids located at the histidine entry and binding sites of ATP-PRT expressed from the *E.coli* hisG were analyzed. The results of the simulation showed that H232, S288, T252, R250, A248, E271 and E240 of hisG are highly likely to interact with histidine (see FIG. 2) .

Based on the results of amino acid mutation analysis and docking analysis of ATP-PRT (hisG) in the mutant strains having increased resistance to TRA, 14 amino acid mutations (H232T, H232E, H232K, E240K, A248F, R250H, R250E, T252A, T252L, T252P, T252Q, E271K, S288K, and S288P) having a high likelihood of increasing histidine production due to a reduction in histidine-induced negative feedback were selected as candidates.

### Example 3: Construction of strain expressing ATP-PRT variant having one mutation and evaluation of histidine productivity of the strain

A one-step inactivation method was used to introduce the point mutated hisG_H232K into the chromosome of the *E*. *coli* DS9H strain (Warner et al., PNAS, 6:6640-6645 (2000)). First, in order to obtain the forward and reverse fragments of hisG gene for homologous recombination, hisG_HF and hisG_HR fragments were amplified using *E. coli* DS9H genomic DNA as a template and the primer pairs hisG_HF-F/hisG_HF-R and hisG_HR-F/hisG_HR-R, respectively. Then, a cassette fragment containing a kanamycin antibiotic marker and FRT was obtained by amplification from a pKD13 plasmid using FR(hisG)-F/FR(hisG)-R. Finally, to obtain hisG_H232K, two fragments were obtained from *E. coli* DS9H genomic DNA using the primer pairs hisG+FR-F/232K-R and 232K-F/hisG+HR-R, respectively. The two obtained fragments were ligated into one fragment using the hisG+FR-F/hisG+HR-R primers to obtain a hisG_H232K fragment. The four finally amplified PCR fragments as templates were ligated into one fragment by overlapping PCR using the primer pair hisG_HF-F/hisG_HR-R. The ligated DNA fragment was introduced by electroporation into an *E. coli* DS9H strain containing a pKD46 plasmid. Thereafter, the kanamycin-resistant cell lines were subjected to PCR using the hisGW-CF/hisGW-CR primers, thereby identifying strains into which hisG_H232K has been introduced. A process of removing the kanamycin marker, which is an antibiotic resistance gene, was performed on the strains confirmed to have introduced hisG_H232K. After inducing FLP recombination by introducing a pCP20 plasmid into the strains confirmed to have introduced hisG_H232K, the removal of the antibiotic was confirmed by checking whether the strains grew on LB plate media containing and not containing the antibiotic (kanamycin), respectively. Here, the confirmation was based on the fact that the strains from which the antibiotic has been removed grew on the LB plate medium, but did not grow on the LB plate medium containing the antibiotic (kanamycin). Finally, the sequences were analyzed using the primer pair hisGW-CF/hisGW-CR. Each of hisG_H232T, hisG_R250H, hisG_T252A, hisG_T252L, hisG_E271K, hisG_S288P, hisG_H232E, hisG_240K, hisG_A248F, hisG_R250E, hisG_T252P, hisG_T252Q, and hisG_S288K was introduced into the *E. coli* DS9H strain in the same manner as described above.

The primers used in the experiment are shown in Table 3 below.

**[Table 3]**

| SEQ ID NO. | Primer | Nucleotide sequence (5'→3') |
|---|---|---|
| 9 | hisGW-CF | AGTTCATTGTACAATGATGAGCG |
| 10 | hisGW-CR | AGCCGCCAGGAATATACAAC |
| 11 | 232K-R | TTTCATCATGATGTATTTTGATTCGCGC |
| 12 | 232K-F | GCGCGAATCAAAATACATCATGATGAAA |
| 13 | 232E-R | TTCCATCATGATGTATTTTGATTCGCGC |
| 14 | 232E-F | GCGCGAATCAAAATACATCATGATGGAA |
| 15 | 240K-R | TTTATCCAGACGTTCGGTCGGT |
| 16 | 240K-F | ACCGACCGAACGTCTGGATAAA |
| 17 | 248F-R | GAAACCTGGCAGCAGGGCGA |
| 18 | 248F-F | TCGCCCTGCTGCCAGGTTTC |
| 19 | 252A-R | CCCGCCAGCGGCAGAATCGC |
| 20 | 252A-F | GCGATTCTGCCGCTGGCGGG |
| 21 | 250H-R | CCGCCAGCGGCAGAATAGTTGGATG |
| 22 | 250H-F | CATCCAACTATTCTGCCGCTGGCGG |
| 23 | 250E-R | CCGCCAGCGGCAGAATAGTTGGTTC |
| 24 | 250E-F | GAACCAACTATTCTGCCGCTGGCGG |
| 25 | 252L-R | CCGCCAGCGGCAGAATCAATGGGCG |
| 26 | 252L-F | CGCCCATTGATTCTGCCGCTGGCGG |
| 27 | 252P-R | CCGCCAGCGGCAGAATCGGTGGGCG |
| 28 | 252P-F | CGCCCACCGATTCTGCCGCTGGCGG |
| 29 | 252Q-R | CCGCCAGCGGCAGAATCTGTGGGCG |
| 30 | 252Q-F | CGCCCACAGATTCTGCCGCTGGCGG |
| 31 | 271K-R | TTTGCTGCTGACCATGTGCA |
| 32 | 271K-F | TGCACATGGTCAGCAGCAAA |
| 33 | 288P-R | CGGACTGGCACCCAGCGCTTTCA |
| 34 | 288P-F | TGAAAGCGCTGGGTGCCAGTCCG |
| 35 | 288K-R | CTTACTGGCACCCAGCGCTTTCA |
| 36 | 288K-F | TGAAAGCGCTGGGTGCCAGTAAG |
| 37 | 232T-R | TGTCATCATGATGTATTTTGATTCGCGC |
| 38 | 232T-F | GCGCGAATCAAAATACATCATGATGACA |
| 39 | hisG_HF-F | GCTCATTCATTAAACAAATCCATTGC |
| 40 | hisG_HF-R | TTTGTTATTCCTCTTTAAACCTGTC |
| 41 | FR(hisG)-F | GTTTAAAGAGGAATAACAAAGTGTAGGCTGGAGCTGCTTC |
| 42 | FR(hisG)-R | CCAGATCAATTCGCGCTAACTCTGTCAAACATGAGAATTAA |
| 43 | hisG+FR-F | TTAATTCTCATGTTTGACAGAGTTAGCGCGAATTGATCTGG |
| 44 | hisG+HR-R | TGTGTTAAAGCTCATGGCGATCACTCCATCATCTTCTCAATCG |
| 45 | hisG_HR-F | TCGCCATGAGCTTTAACACAA |
| 46 | hisG_HR-R | AGTGTGGAAGGTTTCAATATTCTT |

Referring to Table 4 below, histidine production in the strain into which hisG_H232K or hisG_H232T has been introduced increased by about 22% to 26% compared to that in the control group. Histidine production in the strain into which hisG_T252A or T252L has been introduced increased by about 35% to 39% compared to that in the control group. Histidine production in the strain into which hisG_E271K has been introduced increased by about 34% compared to that in the control group. In particular, histidine production in the strain into which hisG_S288P has been introduced increased by about 46% compared to that in the control group, and histidine production in the hisG_R250H-introduced strain increased by about 67%, which is the highest increase, compared to that in the control group.

However, histidine production in the case of the H232E, E240K, and A248F mutations decreased rather than increased, and histidine production in the case of the R250E, T252P, T252Q, and S288K mutations did not significantly increase.

**[Table 4]**

| Strain name | L-histidine (%) | Culture time (hr) | Histidine production |
|---|---|---|---|
| DS9H | 0.78 | 72 | - |
| DS9H_△hisG: :hisG_H232K | 0.95 | 72 | Increased |
| DS9H_△hisG: :hisG_H232T | 0.99 | 72 | Increased |
| DS9H_△hisG: :hisG_R250H | 1.31 | 72 | Increased |
| DS9H_△hisG: :hisG_T252A | 1.06 | 72 | Increased |
| DS9H_△hisG: :hisG_T252L | 1.09 | 72 | Increased |
| DS9H_△hisG: :hisG_E271K | 1.05 | 72 | Increased |
| DS9H_△hisG: :hisG_S288P | 1.14 | 72 | Increased |
| DS9H_△hisG: :hisG_H232E | 0.74 | 72 | Decreased |
| DS9H_△hisG: :hisG_E240K | 0.69 | 72 | Decreased |
| DS9H_△hisG: :hisG_A248F | 0.65 | 72 | Decreased |
| DS9H_△hisG: :hisG_R250E | 0.82 | 72 | No significant change |
| DS9H_△hisG: :hisG_T252P | 0.79 | 72 | No significant change |
| DS9H_△hisG: :hisG_T252Q | 0.77 | 72 | No significant change |
| DS9H_△hisG: :hisG_S288K | 0.80 | 72 | No significant change |

Referring to the above results, the seven mutations (H232T, H232K, R250H, T252A, T252L, E271K, and S288P) increased histidine production. This is believed to be because feedback inhibition by histidine was reduced. Next, the present inventors examined whether histidine production could be further increased by combining these mutations.

### Example 4: Construction of plasmid into which hisG_SDM4 (H232K, T252A, E271K, and S288P) has been introduced

By performing overlapping PCR, the present inventors constructed a plasmid capable of expressing an *E. coli* hisG-derived ATP-PRT enzyme variant containing amino acid substitutions of H232K, T252A, E271K, and S288P. First, genes were amplified with Pfu PreMix (Bioneer) using three primer pairs (hisG-F/232K-R, 232K-F/252A-R, and 252A-F/hisG-R), respectively. Then, the three amplified fragments were ligated into one fragment (which hereinafter may be referred to as the SDM3 fragment) by performing PCR once more using the three fragments as templates and the primer pair hisG-F/hisG-R. Then, each of the SDM3 fragment and a pTRC99A plasmid was treated with the restriction enzymes EcoRI and HindIII (NEB), and the SDM3 fragment was introduced into the pTRC99A plasmid using T4 ligase to obtain pTRC99A-hisG_SDM3. PCR was performed using pTRC99A-hisG_SDM3 as a template and the primer pair hisG-F/271K-R2 to obtain an SDM4 fragment into which four mutations (H232K, T252A, E271K, and S288P) have been introduced.

Then, the SDM4 fragment and a pTRC99A-hisG_SDM3 plasmid were each treated with the restriction enzymes EcoRI and AfeI (NEB) and were ligated together using T4 ligase (Takara), thereby constructing pTRC99A-hisG_SDM4. Finally, the sequence was analyzed using the primer pair hisG-CF/hisG-CR (see Table 5 below). The ATP-PRT variant containing H232K, T252A, E271K and S288P mutations was named hisG_SDM4.

**[Table 5]**

| SEQ ID NO. | Primer | Nucleotide sequence (5'→3') |
|---|---|---|
| 47 | hisG-F | ATATGAATTCATGACAGACAACACTCGTTTACG |
| 48 | hisG-R | ATATAAGCTTTCACTCCATCATCTTCTCAATCGGCAGGACCAGAATCGG |
| 11 | 232K-R | TTTCATCATGATGTATTTTGATTCGCGC |
| 12 | 232K-F | GCGCGAATCAAAATACATCATGATGAAA |
| 19 | 252A-R | CCCGCCAGCGGCAGAATCGC |
| 20 | 252A-F | GCGATTCTGCCGCTGGCGGG |
| 49 | 271K-R2 | ATATAGCGCTTTCAGTTTTTCCATGGTTTCCCAGAACAGGGTTTT |
| 50 | hisG-CF | ATATTCTGAAATGAGCTGTTGACAA |
| 51 | hisG-CR | TACTGCCGCCAGGCAAATTC |

### Example 5: Construction of plasmid into which hisG_SDM7 (H232T, R250H, T252L, E271K, and S288P) has been introduced

A part of the amino acid sequence of the enzyme hisG_SDM4 was substituted with other amino acids to obtain hisG_SDM7 which was then introduced into a plasmid. pTRC99A-hisG_SDM4 was used as a template, and the amino acid at position 232 was substituted with T, the amino acid at position 250 with H, and the amino acid at position 252 with L. Two mutations (E271K and S288P) were maintained. (Compared to hisG_WT, the positions of mutations in hisG_SDM7 are H232T, R250H, T252L, E271K, and S288P).

First, genes were amplified with Pfu PreMix (Bioneer) using three primer pairs (hisG-F/232T-R, 232T-F/250H+252L-R, and 250H+252L-F/hisG-R), respectively. Then, the three amplified fragments were ligated into one fragment by performing PCR once more by using the three fragments as templates and the primer pair hisG-F/hisG-R. Then, the PCR fragment and a pTRC99A plasmid were each digested with EcoRI and HindIII (NEB) and were ligated together using T4 ligase (Takara), thereby constructing pTRC99A-hisG_SDM7. Finally, the sequence was analyzed using hisG-CF/hisG-CR primers (see Table 6 below). The ATP-PRT variant containing H232T, R250H, T252L, E271K and S288P mutations was named hisG_SDM7.

**[Table 6]**

| SEQ ID NO. | Primer | Nucleotide sequence (5'→3') |
|---|---|---|
| 47 | hisG-F | ATATGAATTCATGACAGACAACACTCGTTTACG |
| 48 | hisG-R | ATATAAGCTTTCACTCCATCATCTTCTCAATCGGCAGGACCAGAATCGG |
| 37 | 232T-R | TGTCATCATGATGTATTTTGATTCGCGC |
| 38 | 232T-F | GCGCGAATCAAAATACATCATGATGACA |
| 50 | hisG-CF | ATATTCTGAAATGAGCTGTTGACAA |
| 51 | hisG-CR | TACTGCCGCCAGGCAAATTC |
| 52 | 250H+252L-R | CCGCCAGCGGCAGAATCAATGGATG |
| 53 | 250H+252L-F | CATCCATTGATTCTGCCGCTGGCGG |

### Example 6: Construction of mutant strain into which hisG_SDM4 or hisG_SDM7 gene has been introduced

### 6-1. Construction of mutant strain into which hisG_SDM4 gene has been introduced

A one-step inactivation method was used to introduce hisG_SDM4 into the chromosome of the *E. coli* DS9H strain (Warner et al., PNAS, 6:6640-6645 (2000)). First, in order to obtain the forward and reverse fragments of hisG gene for homologous recombination, hisG_HF and hisG_HR fragments were amplified using *E. coli* DS9H genomic DNA as a template and the primer pairs hisG_HF-F/hisG_HF-R and hisG_HR-F/hisG_HR-R, respectively. Then, a cassette fragment containing a kanamycin antibiotic marker and FRT was obtained by amplification from a pKD13 plasmid using FR(hisG)-F/FR(hisG)-R. Finally, a hisG_SDM4 fragment was obtained from a pTRC99A-hisG_SDM4 plasmid using hisG+FR-F/hisG+HR-R primers. The four finally amplified PCR fragments as templates were ligated into one fragment by overlapping PCR using the primer pair hisG_HF-F/hisG_HR-R. The ligated DNA fragment was introduced by electroporation into an *E. coli* DS9H strain containing a pKD46 plasmid. Thereafter, the kanamycin-resistant cell lines were subjected to PCR using hisGW-CF/hisGW-CR primers, thereby identifying strains into which hisG_SDM4 has been introduced. A process of removing the kanamycin marker, which is an antibiotic resistance gene, was performed on the strains confirmed to have introduced hisG_SDM4. After inducing FLP recombination by introducing a pCP20 plasmid into the strains confirmed to have introduced hisG_SDM4, the removal of the antibiotic was confirmed by checking growth on LB plate media containing and not containing the antibiotic (kanamycin), respectively. Here, the confirmation was based on the fact that the strains from which the antibiotic has been removed grew on the LB plate medium, but did not grow on the LB plate medium containing the antibiotic (kanamycin). Finally, the sequence was analyzed using the primer pair hisGW-CF/hisGW-CR. The primers used in the experiment are shown in Table 7 below.

**[Table 7]**

| SEQ ID NO. | Primer | Nucleotide sequence (5'→3') |
|---|---|---|
| 39 | hisG_HF-F | GCTCATTCATTAAACAAATCCATTGC |
| 40 | hisG_HF-R | TTTGTTATTCCTCTTTAAACCTGTC |
| 41 | FR(hisG)-F | GTTTAAAGAGGAATAACAAA GTGTAGGCTGGAGCTGCTTC |
| 42 | FR(hisG)-R | CCAGATCAATTCGCGCTAACTCTGTCAAACATGAGAATTAA |
| 43 | hisG+FR-F | TTAATTCTCATGTTTGACAGAGTTAGCGCGAATTGATCTGG |
| 44 | hisG+HR-R | TGTGTTAAAGCTCATGGCGATCACTCCATCATCTTCTCAATCG |
| 45 | hisG_HR-F | TCGCCATGAGCTTTAACACAA |
| 46 | hisG_HR-R | AGTGTGGAAGGTTTCAATATTCTT |
| 9 | hisGW-CF | AGTTCATTGTACAATGATGAGCG |
| 10 | hisGW-CR | AGCCGCCAGGAATATACAAC |

### 6-2. Construction of mutant strain into which hisG_SDM7 gene has been introduced

A one-step inactivation method was used to introduce hisG_SDM7 into the chromosome of the *E. coli* DS9H strain (Warner et al., PNAS, 6:6640-6645 (2000)). First, in order to obtain the forward and reverse fragments of hisG gene for homologous recombination, hisG_HF and hisG_HR fragments were amplified using *E. coli* DS9H genomic DNA as a template and the primer pairs hisG_HF-F/hisG_HF-R and hisG_HR-F/hisG_HR-R, respectively. Then, a cassette fragment containing a kanamycin antibiotic marker and FRT was obtained by amplification from a pKD13 plasmid using FR(hisG)-F/FR(hisG)-R. Finally, a hisG_SDM7 fragment was obtained from a pTRC99A-hisG_SDM7 plasmid using hisG+FR-F/hisG+HR-R primers. The four finally amplified PCR fragments as templates were ligated into one fragment by overlapping PCR using the primer pair hisG_HF-F/hisG_HR-R. The ligated DNA fragment was introduced by electroporation into an *E. coli* DS9H strain containing a pKD46 plasmid. Thereafter, the kanamycin-resistant cell lines were subjected to PCR using hisGW-CF/hisGW-CR primers, thereby identifying strains into which hisG_SDM7 has been introduced. A process of removing the kanamycin marker, which is an antibiotic resistance gene, was performed on the strains confirmed to have introduced hisG_SDM7. After inducing FLP recombination by introducing a pCP20 plasmid into the strains confirmed to have introduced hisG_SDM7, the removal of the antibiotic was confirmed by checking growth on LB plate media containing and not containing the antibiotic (kanamycin), respectively. Here, the confirmation was based on the fact that the strains from which the antibiotic has been removed grew on the LB plate medium, but did not grow on the LB plate medium containing the antibiotic (kanamycin). Finally, the sequence was analyzed using the primer pair hisGW-CF/hisGW-CR. The primers used for the construction of the mutant strain into which the hisG_SDM7 gene has been introduced are shown in Table 6 above.

### Example 7: Measurement of resistance of mutant enzyme, expressed from hisG_SDM4 or hisG_SDM7 gene, to negative feedback by histidine

The resistances of the ATP-PRT wild-type (hisG_WT) and the ATP-PRT variants (hisG_SDM4 and hisG_SDM7) to negative feedback by histidine were compared.

50 ml of LB medium was dispensed into each 500-ml flask and inoculated with each of three strains (DS9H, DS9H_△hisG: :hisG_SDM4, and DS9H_△hisG: :hisG_SDM7) at 1%. Each strain was cultured under conditions of 30°C and 180 rpm. When the OD₆₀₀ reached 0.6, ATP-PRT expression was induced with 1 mM IPTG (final concentration) and additional culture was performed for about 4 hours. The cells obtained after culturing were sonicated and centrifuged. The obtained supernatants were used for evaluation of ATP phosphoribosyltransferase activity. The reaction for evaluating the enzyme activity was carried out with reference to existing literature (Microb Cell Fact. 2018. Mar.17:42). The protein in each supernatant was quantified to match the concentration, and the enzyme activity was measured after mixing the reactants according to the reaction composition shown in Table 8 below.

**[Table 8]**

| Component | Concentration |
|---|---|
| Tris-HCl (pH 8.1) | 100 mM |
| Potassium chloride | 100 mM |
| Magnesium chloride | 10 mM |
| ATP | 5 mM |
| PRPP | 1 mM |
| Pyrophosphatase | 10 mU |
| ATP phosphoribosyltransferase | 500 nM |
| Histidine | 0 mM, 0.5 mM, 1 mM, 5 mM, 10 mM, 25 mM, or 50 mM |

In particular, in order to examine resistance to activity inhibition by histidine, the concentrations of histidine were set to 0 mM, 0.5 mM, 1 mM, 5 mM, 10 mM, 25 mM, and 50 mM, respectively. The activity was measured at 30°C at a UV wavelength of 290 nm at 2 minute intervals for 30 minutes.

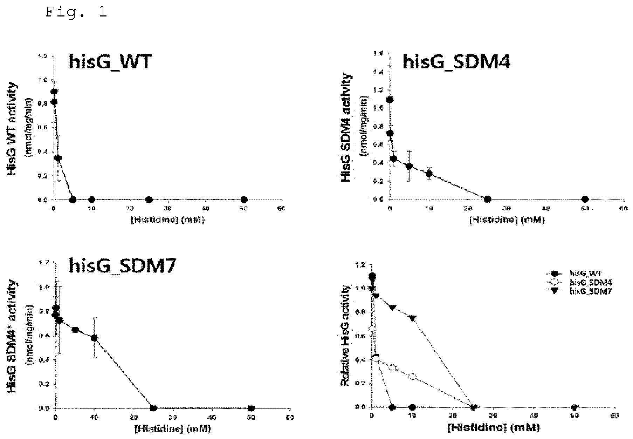

Referring to FIG. 1, the ATP-PRT activity of the hisG_WT enzyme decreased rapidly from a histidine concentration of 5 mM. However, the enzyme activity of hisG_SDM4 (H232K, T252A, E271K, and S288P) decreased from a histidine concentration of 25 mM. In addition, the enzyme activity of hisG_SDM7 (H232T, R250H, T252L, E271K, and S288P) decreased from a histidine concentration of 25 mM, similar to that of hisG_SDM4, but the enzyme activity thereof at each histidine concentration increased compared to that of hisG_SDM4. As a result, hisG_SDM7 had the best resistance to activity inhibition by histidine.

### Example 8: Evaluation of histidine productivities of strains expressing ATP-PRT mutant enzymes

The present inventors evaluated the histidine productivities of the strains into which hisG_SDM4 or hisG_SDM7 has been introduced. 10 ml of a medium having the composition shown in Table 9 below was dispensed into each flask and inoculated with the DS9H, DS9H_△hisG::hisG_SDM4, or DS9H_△hisG: :hisG_SDM7 strain at 1%, and each strain was cultured under conditions of 34°C and 200 rpm for 72 hours. After culturing, the histidine production in each flask was comparatively analyzed.

**[Table 9]**

| Component | Concentration |
|---|---|
| Glucose | 8% |
| Magnesium sulfate | 0.1% |
| Ammonium sulfate | 2.0% |
| MSG | 0.1% |
| Potassium phosphate monobasic | 0.1% |
| Yeast extract | 0.1% |
| Potassium sulfate | 0.02% |
| Thiamine-HCl | 20 ppm |
| Nicotinic acid | 10 ppm |
| Iron sulfate | 5 ppm |
| Zinc sulfate | 5 ppm |
| Manganese sulfate | 5 ppm |
| Calcium carbonate (separately sterilized) | 5.0% |

Histidine production in the hisG_SDM4-expressing strain increased by about 53% compared to that in the control group, and histidine production in the hisG_SDM7-expressing strain increased by about 92% compared to that in the control group (see Table 10 below).

**[Table 10]**

| Strain name | L-histidine (%) | Culture time (hr) |
|---|---|---|
| DS9H | 0.78 | 72 |
| DS9H_△hisG::hisG_SDM4 (H232K, T252A, E271K, and S288P) | 1.20 | 72 |
| DS9H_△hisG::hisG_SDM7 (H232T, R250H, T252L, E271K, and S288P) | 1.50 | 72 |

Referring to the above results, it is thought that the histidine productivity of the strain expressing hisG_SDM4 or hisG_SDM7 increased because feedback inhibition of hisG_SDM4 or hisG_SDM7 by histidine decreased compared to his_WT. In particular, the histidine productivity of the strain expressing hisG_SDM7 was higher than that of the strain expressing hisG_SDM4.

In addition, taking the results in Table 4 and Table 8 above together, when any one of the amino acids at positions 232, 250, 252, 271 and 288 in *E. coli*-derived hisG was mutated, histidine production increased, and when two or more of these amino acids were mutated, histidine production further increased compared to that when any one amino acid was mutated.

### [Accession Number]

Depository authority: Korea Research Institute of Bioscience and Biotechnology
Accession number: KCTC14419BP
Deposit date: December 28, 2020

## Claims

1. An ATP-phosphoribosyltransferase variant containing a substitution of proline for serine at position 288 in an ATP-phosphoribosyltransferase consisting of the amino acid sequence of SEQ ID NO: 1.

2. The ATP-phosphoribosyltransferase variant of claim 1, wherein the ATP-phosphoribosyltransferase is expressed from *E. coli* hisG gene.

3. The ATP-phosphoribosyltransferase variant of claim 1, wherein the variant is reduced feedback inhibition of the variant by histidine.

4. The ATP-phosphoribosyltransferase variant of claim 1, wherein the variant further contains at least one of the following amino acid substitutions:
(a) a substitution of lysine or threonine for histidine at position 232;
(b) a substitution of histidine for arginine at position 250;
(c) a substitution of alanine, leucine, glycine, valine or isoleucine for threonine at position 252; and
(d) a substitution of lysine for glutamic acid at position 271.

5. A transformed strain expressing the ATP-phosphoribosyltransferase variant of claim 1.

6. The transformed strain of claim 5, wherein the strain is *E. coli.*

7. A method for producing histidine comprising a step of culturing the strain of claim 5.
